Europäisches Patentamt

European Patent Office

Office européen des brevets

(19)

(11) Publication number: **0 142 345**
**A2**

(12) # EUROPEAN PATENT APPLICATION

(21) Application number: **84307750.4**

(22) Date of filing: **09.11.84**

(51) Int. Cl.⁴: **A 61 K 39/395**

(30) Priority: **09.11.83 US 549966**

(43) Date of publication of application:
**22.05.85 Bulletin 85/21**

(84) Designated Contracting States:
**AT BE CH DE FR GB IT LI LU NL SE**

(71) Applicant: **SYNBIOTICS CORPORATION**
**11011 Via Frontera**
**San Diego California 92127(US)**

(72) Inventor: **Maggio, Edward T.**
**12588 Chetenham Lane**
**San Diego California 92128(US)**

(74) Representative: **Hedley, Nicholas James**
**Matthew et al,**
**T.Z. Gold & Company 9 Staple Inn**
**London WC1V 7QH(GB)**

(54) Anti-idiotypic vaccine employing anti-idiotypic monoclonal antibodies.

(57) Anti-idiotypic vaccines and methods for making anti-idiotypic vaccines are described which employ a combination of anti-idiotypic monoclonal antibodies emulsified with an adjuvant. When administered to an immune responsive subject, the anti-idiotypic vaccine may elicit an immune response which includes the immunogenic production of an array of idiotypic antibodies directed against a designated infectious disease organism or other pathogenic agent. Each anti-idiotypic monoclonal antibody within the anti-idiotypic vaccine is developed through a multistep process which begins by challenging immune responsive subjects with antigen from an infectious disease organism or other pathogenic agent. A variety of selection steps occur for idiotypic monoclonal antibody and for anti-idiotypic monoclonal antibody both before and after the hybridoma expansion for each. The selected anti-idiotypic monoclonal antibody has a variable region that defines its antigen binding site which displays a structural conguence with at least one epitope found on the infectious disease organism or pathogenic agent. The antigenicity of this antigen binding site is similar to the antigenicity of the epitope found on the infectious disease organism or other pathogenic agent. Each anti-idiotypic monoclonal antibody may be fully characterized as to its antigenic potential, its immunogenic specificity, and the neutralizing efficacy of its immune response. The selection and compounding of useful anti-idiotypic monoc- lonal antibody within the anti-idiotypic vaccine and the dosage optimization of each component enhances the clinical utility of the anti-idiotypic vaccine. In order to minimize zenogenic effects, the anti-idiotypic vaccine may include Fab fragments of the anti-idiotypic monoclonal antibodies. Methods are described for producing and selecting the component anti-idiotypic monoclonal antibodies from idiotypic monoclonal antibodies directed against the designated infectious disease organism or other pathogenic agent. Methods are described for using the anti-idiotypic vaccines to impart either active or passive immunity.

ANTI-IDIOTYPIC VACCINE EMPLOYING

ANTI-IDIOTYPIC MONOCLONAL ANTIBODIES


SPECIFICATIONS


Background of the Invention

The invention relates to anti-idiotypic vaccines, to methods for the manufacture of anti-idiotypic vaccines, and to methods for the utilization of anti-idiotypic vaccines. More specifically, the invention relates to anti-idiotypic vaccines having a composition which includes monoclonal anti-idiotypic antibodies or fragments thereof.

Anti-idiotypic vaccines are compositions which elicit an immune response, including the production of antibody, when administered to an immune responsive subject. Anti-idiotypic vaccines are used clinically to elicit an immune response which causes the subject to become resistant to an infectious disease organism.

In the past, anti-idiotypic vaccines consisted of killed infectious disease organisms, attenuated infectious disease organisms (live), or extracts obtained from such organisms. Such vaccines have been highly successful in suppressing or eliminating such diseases as small pox, diphtheria, rubella, polio, and others. Although successful, such vaccines suffer from a number of serious shortcomings, viz.:

1.) An attenuated strain, used as a vaccine, has an ever present danger of spontaneously reverting to a virulent form of the infectious disease organism.

2.) During the cultivation process of disease organisms, there is an ever present risk of contamination of the vaccine strain by other pathogenic strains, undetected and unattenuated.

3.) The cultivation process of disease organisms in tissue culture or chicken embryo bears an inherent risk of inadvertant contamination by foreign proteins emanating from the culture milieu, unrelated to the disease organism, which are carried into the vaccine.

4.) Certain infectious disease organisms are incapable of satisfactory growth outside of their natural host.

One approach to overcome some of the shortcomings of vaccines was to develop an antigenic preparation composed of chemically synthesized portions of the immunogenic protein molecules. This method was recently described and reviewed by Sutcliffe in Science, Vol. 219, pp 660-666, 1983. Antigenic portions of immunogenic protein molecules were determined, sequenced, and chemically synthesized. Antigenic preparations composed of chemically synthesized immunogenic subunits are then used to immunize subject animals. These immunized animals are then screened firstly to determine if they produce antiserum to the infectious disease organism and secondly to determine if this antiserum can neutralize the infectious disease organism. Sutcliffe demonstrated that antigenic preparations composed of chemically synthesized peptide subunits can sometimes be used effectively. However, Sutcliffe's method suffers certain inherent limitations:

1.) Before the subunits can be synthesized, the antigenic portions of immunogenic proteins must be identified and sequenced. The tasks of identification, sequencing, and synthesis are time consuming, laborious and expensive. Also, the molecular biology of many infectious disease organisms is poorly characterized and must be worked out prior to development of the synthetic immunogenic subunits.

2.) The conformation of an antigenic site found in a native protein may frequently be difficult to duplicate in a short synthetic peptide. The conformation of a native protein frequently depends upon secondary and tertiary structural features which develop during biosynthesis. An antigenic site having an energetically unfavorable conformation within a conformationally stable protein will probably be difficult to duplicate using a short synthetic peptide which lacks the larger secondary and tertiary structural features which might support such an unfavorable conformation.

3.) Antigenic determinants arising from post-transcriptional alterations, such as an addition of glycosyl residues to the side chains of the peptide sequence, are not easily duplicated using this synthetic subunit approach.

Other alternatives to vaccines include anti-idiotypic vaccines composed of anti-idiotypic polyclonal antibodies. Such anti-idiotypic vaccines were recently described by Sacks in the Journal of Experimental Medicine, Vol. 155, pp 1108-1119, 1982. Sacks first developed three different idiotypic monoclonal antibodies having a specificity directed against an infectious organism. Then, each idiotypic monoclonal antibody was injected into an animal to raise antiserum against that particular idiotypic antibody. Anti-idiotypic polyclonal antibodies were then isolated from this antiserum. Each anti-idiotypic polyclonal antibody was shown to bind its respective idiotypic monoclonal antibody. Challenge by anti-idiotypic polyclonal antibodies conferred an immunity, or partial immunity, to the disease organism within a substantial fraction of test animals. The best results were obtained when all three types of anti-idiotypic polyclonal antibodies were pooled and injected into the test animal. Sacks' preparation is distinguished from the present invention because Sacks used anti-idiotypic polyclonal antibody instead of anti-idiotypic monoclonal antibody. Specifically, Sacks failed to teach that anti-idiotypic monoclonal antibody can confer an immunity; that each anti-idiotypic monoclonal antibody within a battery can be individually screened and selected for its ability to elicit the immune production of idiotypic antibodies directed against target antigen; that the antigenic potential of each anti-idiotypic monoclonal antibody within a battery can be characterized individually and its optimal dosage determined; that each anti-idiotypic monoclonal antibody within a battery can be screened to determine which elicits an antiserum that most effectively neutralizes the disease organism; that a battery of screened anti-idiotypic monoclonal antibodies would be highly advantageous over unscreened anti-idiotypic polyclonal antibodies; or that anti-idiotypic antibody fragments can be used in lieu of a whole antibody and that such fragments are more focused in their antigenic potential against the disease organism.

Prior to Sacks work, Kelsoe taught that anti-idiotypic monoclonal antibodies could be used to regulate idiotypic expression, Immunological Review, Vol. 52, pp 75-88, 1980. Kelsoe showed that pretreatment of a subject with an anti-idiotypic monoclonal antibody, having a specificity against an idiotype, could regulate the expression of that idiotype when the subject was subsequently challenged by a antigen which was known to stimulate the production of that idiotype. Pretreatment could either stimulate or suppress subsequent expression of idiotype, depending on the dosage and timing. However, like Sacks, Kelsoe did not teach that anti-idiotypic monoclonal antibody alone was sufficient to stimulate the immune production of idiotypic antibody.

## Summary of the Invention

The invention is a type of anti-idiotypic vaccine, methods for making such anti-idiotypic vaccines, and methods for utilizing such anti-idiotypic vaccines. The anti-idiotypic vaccine is capable of eliciting a specific immune response directed against infectious disease organisms or other pathogenic agents when administered to an immune responsive subject. The subject's immune response to the anti-idiotypic vaccine includes the immunogenic production of antibodies which bind to antigenic sites on the infectious disease organism or pathogenic agent. More particularly, the anti-idiotypic vaccine may be adapted to elicit an immune response which selectively produces idiotypes having a binding specificity and avidity for specific antigenic sites; which selectively produces idiotypes having a binding specificity for agglutinogens found on the disease organism or pathogenic agent, which binding specificity enables the idiotype to participate in agglutination reactions with the disease organism, pathogenic agent, or other model agglutinable bodies; which selectively produces idiotypes which are shown to be effective in neutralizing the infectious disease organism or other pathogenic agent; and which produces minimal amounts of undesirable idiotypes.

In the initial step for making the anti-idiotypic vaccine, an immune responsive subject is challenged with antigen from the infectious disease organism or other pathogenic agent in order to stimulate the immunogenic production of lymphocytes which produce idiotypic antibody.

These lymphocytes are then used to produce hybridomas which are subsequently screened and selected for their production of useful idiotypic antibody. Idiotypic monoclonal antibody is then generated in quantity by the selected hybridoma and purified. Purified idiotypic monoclonal antibody is then injected into a second immune responsive subject in order to stimulate the immunogenic production of lymphocytes which produce anti-idiotypic antibody. These lymphocytes are then used to generate hybridomas, which are subsequently screened and selected for utility of their anti-idiotypic antibody. Selected hybridomas are then used to produce expanded quantities of the selected anti-idiotypic monoclonal antibody, which are then purified.

The process of immunogenesis causes each antibody to have a unique antigen binding site. In the case of an idiotypic monoclonal antibody and its corresponding anti-idiotypic monoclonal antibody, each has a unique antigen binding site which can serve as a template for the immunogenic production of the other. The two antigen binding sites are complementary to one another. On the other hand, the antigen binding site of the anti-idiotypic monoclonal antibody will exhibit a structural congruence with at least one epitope of an antigenic site found on the infectious disease organism or pathogenic agent which stimulated the immunogenic production of the corresponding idiotypic antibody.

Each of the anti-idiotypic monoclonal antibodies are then tested to determine its antigenic potential. The anti-idiotypic monoclonal antibody to be tested is mixed in varying amounts with a carrier fluid or with an adjuvant to form the anti-idiotypic vaccine. These various dosages of anti-idiotypic vaccine are then administered to immune responsive subjects. The dosage yielding the optimal immune response is considered to be the optimal dosage. An optimal immune response includes the optimal immunogenic production of idiotypic antibody. Anti-idiotypic vaccines having a low optimal dosage have a greater antigenic potential than anti-idiotypic vaccines having a high optimal dosage.

Anti-idiotypic vaccines having a useful antigenic potential may be combined to form a combination anti-idiotypic vaccine. The antigenic potential of each component within the combination can be affected by the presence of the other components. The optimal dosage of each component within the combination may be determined for its ability to stimulate the immunogenic production its corresponding idiotypic antibody. The relative dosages of the different components within the combination anti-idiotypic vaccine are optimized to impart the greatest overall antigenic potential for the combination. Injecting an immune responsive subject with the optimal dosage of a combination anti-idiotypic vaccine will challenge the subject's immune system to produce an array of idiotypic antibodies directed against specific antigens on the disease organism or pathogenic agent, each idiotypic antibody being produced in optimal quantities.

Minimally, the anti-idiotypic vaccine must include at least one anti-idiotypic monoclonal antibody or fragment thereof combined with a carrier fluid. The anti-idiotypic monoclonal antibody must have been screened and selected for its antigenic potential to stimulate the immune system of a challenged subject to produce idiotypic antibodies which bind to antigenic sites on a disease organism or pathogenic agent. The anti-idiotypic monoclonal antibody may also be screened and selected for its specificity and avidity of binding with the elicited idiotypic antibody. The antigenic potential of the anti-idiotypic vaccine should be characterized in order to determine the optimal dosage. One preferred embodiment includes a combination of several anti-idiotypic monoclonal antibodies, each antibody having been screened and selected for its ability to elicit idiotypic antibodies directed against a disease organism or pathogenic agent.

In an other preferred embodiment the anti-idiotypic vaccine includes at least one anti-idiotypic monoclonal antibody selected for its antigenic potential to elicit the immunogenic production of idiotypic antibody directed against agglutinogens on the disease organism or pathogenic agent. The elicited idiotypic antibody should be shown to be capable of binding agglutinogen and to agglutinate the disease organism or pathogenic agent thereby. Preferably, such an anti-idiotypic monoclonal antibody will have been derived from template idiotypic monoclonal antibody which, in turn, had been demonstrated to bind to agglutinogen and to cause agglutination thereby. This preferred embodiment

- 10 -

0142345

may also include more than one such anti-idiotypic monoclonal antibody, each of which is capable of eliciting idiotypes which can bind agglutinogens and which can cause agglutination by such binding.

An other preferred embodiment of the anti-idiotypic vaccine includes at least one anti-idiotypic monoclonal antibody selected for its antigenic potential to elicit immunogenic production of idiotypic antibody which can neutralize an infectious disease organism or pathogenic agent. Such anti-idiotypic monoclonal antibody is screened and selected by following the immune response of test subjects first treated with the anti-idiotypic vaccine and later challenged by innoculation with the disease organism or pathogenic agent. Anti-idiotypic monoclonal antibody is selected which tends to cause the neutralization of the infectious disease organism or pathogenic agent in the test subjects. To facilitate such screening, anti-idiotypic monoclonal antibody should first be prescreened for its ability to elicit the immunogenic production of idiotypic antibody demonstrating a specificity and avidity for binding to agglutinogens and for agglutinating the disease organism or pathogenic agent thereby. This preferred embodiment may also include more that one such anti-idiotypic monoclonal antibody each of which can elicit neutralizing idiotypic antibodies.

An other preferred embodiment of the anti-idiotypic vaccine includes preparations composed of Fab fragments or other fragments of anti-idiotypic monoclonal antibody which retain their antigen binding site. In the case of Fab fragments of anti-idiotypic monoclonal antibody, this
"antigen binding site" is structurally congruent with at
least one epitope of the antigen against which the
anti-idiotypic vaccine is directed. The Fab fragment of
the anti-idiotypic monoclonal antibody serves as a template
for the immunogenic production of the corresponding
idiotypic antibody. Challenge by whole xenogenic
anti-idiotypic antibody can cause a response which includes
the immunogenic production of undesirable antibodies
directed against the anti-idiotypic antibody at sites other
than the "antigen binding site." Challenge by whole
xenogenic anti-idiotypic antibody may cause a subject to
develop an immune reaction against the xenogenic antibody
at sites unrelated to the target antigen. The use of Fab
fragments obviates problems associated with challenge by
xenogenic immunoglobulins, viz., the development of
undesirable immune reactions to the anti-idiotypic vaccine.
This preferred embodiment may also include a combination
of several such anti-idiotypic monoclonal antibody
fragments. Each Fab fragment may be screened and selected
for its ability to elicit the immunogenic production of
idiotypic antibody having a binding specificity and avidity
for antigen; for its ability to elicit the immunogenic
production of idiotypic antibodies which can bind to
agglutinogen and cause agglutination thereby; and for its
ability to elicit the immunogenic production of
neutralizing idiotypic antibodies.

The invention also includes methods for using the
anti-idiotypic vaccines. These methods include either of two alternative therapeutic approaches aimed at the treatment of an infectious disease or other pathogenic condition:

1.) Active immunity in which the anti-idiotypic vaccine is injected directly into the subject to be immunized.

2.) Passive immunity, in which the anti-idiotypic vaccine is administered to a host animal to generate antibodies in the host's serum, and following this, removing the host's antibodies, typically as an antiserum preparation, and administering the antiserum containing the desired antibodies to a recipient subject to passively convey immunological protection upon the recipient subject.

Prior to the use of either method, the optimal dosage of the anti-idiotypic vaccine may first be determined. The ability to optimize the dosage of each component of the present invention is a major advantage over the prior art use of anti-idiotypic polyclonal antibodies. An excess dosage of anti-idiotypic antibody can potentially result in the immune suppression of idiotypic antibody expression. An insufficient dosage of anti-idiotypic antibody can result in a clinically undetectable response. The optimal dosage lies between these two extremes and yields an immune response which is substantially maximal or optimal.

In the present invention, the dosage of each anti-idiotypic monoclonal antibody can be separately optimized. Additionally, each combination of two or more different anti-idiotypic monoclonal antibodies can be tested for synergistic and inhibitory interactions between the different clones. Also, the carrier fluid or adjuvant can be optimized for each anti-idiotypic monoclonal antibody.

The determination of the antigenic potential and the optimal dosage for Sacks' anti-idiotypic vaccine is less satisfactory than the present invention. Because Sacks' preparation is composed of polyclonal anti-idiotypic antibodies, only the over all antigenic potential of each polyclonal anti-idiotype can be determined. For such preparations, it is not practical to separate out each antibody clone of the polyclonal mixture to separately determine the optimal dosage to maximally stimulate antibody production. The immune response to a polyclonal anti-idiotypic vaccine such as Sacks' would presumably be a mixture of suppressed clones, optimized clones, and insufficiently stimulated clones.

It is a purpose of this invention to provide an anti-idiotypic vaccine for challenging an immune responsive subject to immunogenically produce idiotypic antibody directed against a disease organism or pathogenic agent. The anti-idiotypic vaccine includes a carrier fluid and an anti-idiotypic monoclonal antibody which has an antigenic potential to elicit the immunogenic production of idiotypic antibody which can bind to antigenic sites on the disease organism or pathogenic agent. The antigenic potential of this anti-idiotypic monoclonal antibody is determined in order to optimize the suggested dosage for maximizing the immunogenic production of idiotypic antibody.

It is a purpose of this invention to provide such an anti-idiotypic vaccine where the elicited idiotypic antibodies have been selected for their specificity and avidity for binding to antigenic sites found on the disease organism or pathogenic agent; where the elicited idiotypic antibodies have been selected for their specificity and avidity for binding to agglutinogen and for agglutinating the disease organism or pathogenic agent thereby; where the elicited idiotypic antibodies have been selected for their ability to neutralize the disease organism or pathogenic agent.

It is a purpose of this invention to combine two or more such anti-idiotypic monoclonal antibodies to form an anti-idiotypic vaccine and to optimize the dosage of each component.

It is a purpose of this invention to substitute Fab fragments or related fragments of the anti-idiotypic monoclonal antibody for the whole antibody within the anti-idiotypic vaccine.

It is a purpose of this invention to provide methods for making such anti-idiotypic vaccines.

It is a purpose of this invention to provide methods to use such anti-idiotypic vaccines both to elicit active immunity and passive immunity.

Definitions

Antibodies - Specific binding proteins with multiple defined tertiary and quaternary structure.

Antigen - Any substance which binds specifically to an antibody.

Anti-idiotope - A binding epitope with specific
anti-idiotypic properties.

Anti-idiotypic Antibodies - Antibodies themselves are
antigenic and may themselves serve as immunogens.
Antibodies which bind to the hypervariable region of a
subject antibody (i.e., idiotypic region or binding
site of the subject antibody) are called anti-idiotypic
antibodies. Thus the subject antibody's binding site
can bind either to its complementary antigen, or to an
anti-idiotypic antibody. In other words, the
hypervariable region of the anti-idiotypic antibody is
a positive three dimensional structural approximation
of the structure of one or more of the antigen's
epitopes.

Anti-idiotypic Vaccine - an antigenic preparation that is
administered to produce or artificially increase
immunity to a particular disease.

Idiotypic Variations or Idiotypes - Structural differences
exhibited by antibodies within a given class in the
hypervariable regions of their structure; these
idiotypic variations, also called idiotypes, determine
the shape and structure of the binding site, and thus
determine the specificity of the antibody.

Isotypic Variations - Structural variations exhibited by
antibodies in the so-called constant region of their
structure which allows them to be segregated into
classes which include the IgG, IgA, IgM, classes
(so-called isotypes).

Fab or (Fab')2 Fragments - Fragments of antibodies
consisting mainly of the antibody binding site,
frequently prepared by selective proteolysis of whole
antibody molecules.

Monoclonal Antibody - Antibody molecules arising from a
single clone of antibody-producing cells having the
same idiotypic and isotypic structure.

Xenogenic - Externally administered protein or tissue that
has been derived from a species different from the
recipient.

Detailed Description of the Invention

Example A

A Method for Producing an Anti-idiotypic Vaccine against
Entamoeba Histolytica Employing a Mixture of Intact
Anti-idiotypic Monoclonal Antibodies

Step 1.) Production of a battery of idiotypic
monoclonal antibodies, each having a specificity directed
against Entamoeba histolytica:

Immune responsive mice are challenged by inoculation
with an extract of Entamoeba histolytica. The extract may
be obtained by disrupting log phase E. histolytica by
sonication in PBS at 4 degrees Centigrade, to yield a
suspension measuring 0.1 milligrams of total protein per mL
of suspension. The E. histolytica extract is then
emulsified with complete Freund's adjuvant yielding an
emulsion having 50 micrograms of total protein per 200
microliters of emulsion. BALB/c mice can then be challenged by injecting each with 200 microliters of this emulsion. After booster injections of similar composition at 1 week and 3 weeks, the mice are sacrificed, their spleens removed and placed in a 60mm petri dish containing 4ml of serum-free medium. A single suspension of splenocytes is generated by teasing the spleen with forceps. The splenocytes are transferred to a 50mL centrifuge tube and enough medium is added to yield a total volume of 40-45mL. The splenocytes are centrifuged for 10-15 minutes at 800g and the pellet is washed by resuspension and centrifugation and brought up in 10mL media. Murine myeloma cells (e.g., cell line P3X63.Ag8.653) are added to the suspension to yield a mixture of cells having a ratio of 1:6 myeloma cells to splenocytes. Polyethlene glycol (PEG) is melted at 56 degrees Centigrade in a water bath and 0.35mL PEG is added to 0.65mL media at 37 degrees Centigrade. Then, 1.00mL of the PEG solution is added to 10mL of the mixture of cells in a dropwise fashion with stirring, thereby causing fusion between the cells. Fusion is halted by adding 9mL of DMEM, 10% FBS, and washing the cell mixture by centrifugation. The washed cells are then placed into 200mL HAT medium containing 2X10(6) mouse thymocytes/mL to stimulate proliferation of the fused cells. The cell suspension is then dispensed into eight 96 well microtiter plates (25mL/plate) and incubated at 37 degrees Centigrade. After days 10 and 18 of the incubation, the depleted supernatant media is removed from each well of the 96 well microtiter

plates and replaced with fresh media. The depleted supernatant media is assayed for the presence of anti-E. histolytica antibodies to determine which wells contain useful fused cell lines which produce and secrete such antibodies.

The supernatant media may be assayed by an heterogeneous immunoassay. One such heterogeneous immunoassay employs microtiter plates coated with antigen of E. histolytica. E. histolytica extract (supra) is mixed with sodium borate buffer to yield 200 nanograms of total protein per 200 microliters of buffer. Then, 200 microliters of the buffered E. histolytica extract is dispensed into each well of a 96 well microtiter polystyrene plate (e.g., Dynatek (TM)). Allowing the buffered E. histolytica extract to stand in each well for four hours at 37 degrees Centigrade, followed by approximately 14 hours at 4 degrees Centigrade, causes antigenic material from the E. histolytica extract to adsorb to the well. The buffered E. histolytica extract is then decanted from each of the wells. The wells are then washed three times with phosphate buffered saline containing 0.025% tween, so that only firmly adsorbed E. histolytica material will remain. After the microtiter plates are allowed to dry, they may be used as coated microtiter plates in an heterogeneous immunoassay.

To assay the supernatant media for the presence of anti-E. histolytica, 150 microliters of supernatant media is transferred from an incubation well at 10 days and again at 18 days to corresponding wells of a coated microtiter plate. The transferred media is then incubated in the well of the coated microtiter plate for 30 minutes so as to allow anti-E. histolytica to bind to the coated microtiter plate. The transferred media is then decanted from each well and the well is washed three times with phosphate buffered saline. After washing, only anti-E. histolytica antibody will remain bound to the the E. histolytica antigens of the coated microtiter plate.

The presence of bound anti-E. histolytica antibody on the coated microtiter plate is detected by the use of a goat anti-mouse IgG-HRP enzyme conjugate. Horseradish peroxidase (HRP) is coupled to goat anti-mouse IgG according to the method of Nakane (Nakane, P.K., and Kawaio, A.T., Histochem and Cytochem, Vol. 22, 1084 (1974)). The goat anti-mouse IgG-HRP enzyme conjugate binds mouse IgG, including mouse IgG which is bound to the E. histolytica antigens of the coated microtiter plate. After treatment with a reagent of goat anti-mouse IgG-HRP enzyme conjugate, the reagent is decanted from the coated microtiter plates and each well of the coated microtiter plates is washed three times with phospate buffered saline. Washing removes goat anti-mouse IgG-HRP enzyme conjugate which is not immobilized by binding to mouse IgG on the coated microtiter plate. The detection of bound goat anti-mouse IgG-HRP enzyme conjugate may be determined by measurement of HRP activity using a color assay (infra). Supernatant media testing positive with the color assay is an indication that the cell line, from which the supernatant media was drawn, is a potentially useful cell line which can secrete anti-E. histolytica antibody.

A color assay for the measurement of HPR activity may utilize 2,2'-azino-di-(3-ethylbenzthiazoline) sulfonic acid (ABTS). An aqueous solution of ABTS is prepared having a concentration of 2g/80mL. The ABTS solution is stored at 4 degrees Centigrade. A 0.01% solution of hydrogen peroxide is prepared by dilution of 30% peroxide into water. To initiate the measurement of HRP activity, 50 microliters of ABTS and 50 microliters of hydrogen peroxide are added to each well of the coated microtiter plate. The development of color will depend upon the HRP enzyme activity, but is typically observed over 5-30 minutes.

Cell lines which test positive by the HRP color assay may have the ability to secrete useful antibody having a binding specificity for E. histolytica. However, such cell lines can be screened further to assess their relative utility. Supernatant media from color positive cell lines may be screened for its ability to agglutinate cell suspensions of E. histolytica. A small aliquot of the supernatant media is mixed with a cellular suspension of E. histolytica and incubated on a microscope slide. The microscope slide is then examined under a light microscope to observe agglutination of the E. histolytica. Alternatively, the agglutination assay may utilize a suspension of synthetic particles (bodies) having surface bound E. histolytica antigen as a substitute for the

cellular E. histolytica. Supernatant media which tests positive for agglutination is a second indication that the cell line, from which the supernatant media was drawn, secretes useful idiotypic antibody having a binding specificity for E. histolytica. Such a cell line may be expanded as a hybridoma so as to constitute one of a battery of hybridomas which can produce useful idiotypic monoclonal antibodies directed against E. histolytica.

The selected murine hybidoma cell lines are expanded in tissue culture. The hybridoma is incubated at 37 degrees Centigrade with DMEM medium containing 10% fetal bovine serum and a 10% partial pressure of carbon dioxide. The cells are harvested and a major aliquot is frozen in liquid nitrogen for storage. The remainder of the harvested cells may be used for injection into mice for the production of ascitic fluid. The harvested cells to be injected into mice are first washed in serum-free medium and resuspended in serum-free medium or phosphate buffered saline, to yield a cell concentration of 6-10x10(6) cells/mL. BALB/c mice, which were previously primed by a interperitoneal injection of 0.5mL pristane (2,6,10,14-tetramethyl pentadecane), may be injected with a 0.5mL aliquot containing 3-5x10(6) resuspended cells. Ascitic fluid will accumulate in the peritoneal cavity of the mouse. This ascitic fluid may be collected after 7-10 days with an 18 to 20 gauge, 1/2" needle. The collected ascitic fluid is then centrifuged at 2800g for 45 minutes and passed through a 0.45 micrometer filter to separate the immunoglobulin from cellular material and other particulates. The immunoglobulin is then fractionated by precipitation with 50% ammonium sulfate. The immunoglobulin portion is then dialyzed exhaustively against PBS, to yield purified idiotypic monoclonal antibody.

Purified idiotypic monoclonal antibody is obtained from each of the hybridomas which tests positive by the ABTS color test and the agglutination test. These purified idiotypic monoclonal antibodies constitute a battery of such antibodies, each having a binding specificity directed against E. histolytica. Each of these purified idiotypic monoclonal antibodies may be further screened for utility by determining its ability to impart passive immunity to a subject upon direct administration, according to methods reviewed by Potocnjak (Potocnjak, Pedro, et al J. Exp. Med. Vol. 151, 1504-1513 (1980)).

Step 2.) Production of a battery of anti-idiotypic monoclonal antibodies against the monoclonal anti-E. histolytica antibodies:

Anti-idiotypic monoclonal antibody is prepared against each idiotypic monoclonal antibody which constitutes the battery that recognizes the parasite E. histolytica. The idiotypic monoclonal antibody is emulsified with complete Freund's adjuvant so as to yield an emulsion having 50 micrograms of the antibody in 200 microliters of the emulsion. An aliquot of 200 microliters of the emulsion is injected into each of several mice. After booster injections of similar composition at 1 week and 3 weeks, the mice are sacrificed, their spleens removed. Splenocytes from the mouse are then used to produce hybridomas having an ability to secrete anti-idiotypic monoclonal antibodies. The procedure to produce these anti-idiotypic hybridomas is directly analogous to the procedure described above to produce the idiotypic hybridomas. The principal difference between the two procedures is the source of the splenocytes and the screening process.

The screening process to find anti-idiotypic hybridomas tests for the presence of anti-idiotypic antibody. Supernatant media is taken from incubating cell lines and assayed for the presence of anti-idiotypic antibodies using a heterogeneous immunoassay.

A heterogeneous immunoassay for detecting anti-idiotypic antibodies is constructed somewhat differently than the heterogeneous immunoassay decribed above for detecting idiotypic antibodies. The coating on the coated microtiter plates and the HRP enzyme conjugate are each different for the two immunoassays.

A HRP enzyme conjugate is formed with the particular idiotypic monoclonal antibody which was used to raise the cell lines which are to be screened. This idiotypic monoclonal antibody is coupled to horseradish peroxidase (HRP), to form Id-HRP enzyme conjugate according to the method of Nakane (Nakane, P.K., and Kawaio, A.T., Histochem and Cytochem, Vol. 22, 1084 (1974).

Coated microtiter plates for the immunoassay of anti-idiotypic antibody are obtained using material derived from supernatant media taken from the incubating cell lines. Each well of a microtiter polystyrene plate receives an aliquot of 200 microliters of sodium borate buffer and an aliquot of 50 microliters of supernatant media taken from a corresponding incubation well. The buffered mixture is then incubated in the well for four hours at 37 degrees Centigrade and for approximately 14 hours at 4 degrees Centigrade to allow material from the supernatant media to adsorb to the well. The buffered mixture is then decanted from the well. The wells are then washed three times with phosphate buffered saline containing 0.025% tween, so that only adsorbed material remains. After the microtiter plates are allowed to dry, they may be used in an heterogeneous immunoassay.

Each well of the coated microtiter plate has adsorbed material which is derived from the supernatant media taken from a corresponding incubation well. To assay each well of the coated microtiter plate for the presence of useful adsorbed anti-idiotypic antibody, an aliquot of the Id-HRP enzyme conjugate is dispensed into each well. The Id-HRP enzyme conjugate is made up in PBS, pH 7.4. The aliquot dispensed into each well is 150 microliters. The microtiter plates are then incubated for 30 minutes and washed three times with PBS. Those wells coated with anti-idiotypic antibody are found to bind the Id-HRP enzyme conjugate. Binding of Id-HRP produces antibodies which have activity directed against heavy chain allotype, many of the wells may yield a false positive.

To further distinguish useful anti-idiotypic antibody which has a structual congruence with E. histolytica antigen and which binds to the binding site of the idiotypic monoclonal antibody, from antibody having activity against only heavy chain allotype, the immunoassay protocol is then repeated using an antigen blocking procedure. The second immunoassay is identical to the first, except competing antigen (200 micrograms of E. histolytica extract in 25 microliters) is dispensed into each well of the coated microtiter plate directly prior to dispensing the Id-HRP enzyme conjugate into the wells. The Id-HRP enzyme conjugate will bind to the antigen. If the well is coated with anti-idiotypic antibody, the binding between the idiotypic antibody and the antigen will block the binding between anti-idiotypic antibody and the idiotypic antibody and the Id-HRP enzyme conjugate will not bind to the well. However, if the well is coated with antibody having activity only against heavy chain allotype, the Id-HRP enzyme conjugate will still bind to the well. Wells exibiting no Id-HRP binding in the presence of E. histolytica antigen pretreatment, and strong Id-HRP binding in the absence of E. histolytica pretreatment indicate the presence of cells secreting useful anti-idiotypic antibody. Wells which continue to exhibit Id-HRP enzyme conjugate in the presence of the blocking E. histolytica antigen are exposed as false positives.

Cells found to secrete useful anti-idiotypic antibody are expanded in tissue culture. A portion of the cells are frozen in liquid nitrogen for storage. An other portion of the cells are injected into mice for production of ascitic fluid and isolation of anti-idiotypic monoclonal antibody according to the procedure described above for the production of idiotypic monoclonal antibody.

Anti-idiotypic monoclonal antibodies (Anti-Id) are purified from murine ascitic fluid by centrifugation, filtration, fractionation by precipitation with 50% saturated ammonuim sulfate, and exhaustive dialysis against phosphate buffered saline (PBS), in a procedure identical to the purification of idiotypic monoclonal antibody described above.

Although it is preferred to produce the battery of anti-idiotypic monoclonal antibodies using a battery of screened idiotypic monoclonal antibodies, it should be noted that polyclonal idiotypic antibody could be used in place of the battery of idiotypic monoclonal antibodies to produce the anti-idiotypic battery. However, since many useful clonal products may be poorly represented in any given population of polyclonal idiotypic antibodies, such useful clonal products may not have sufficient representation to elicit an immune response when challenging an immune responsive subject. Selecting and expanding desirable idiotypes at the first step increases the chances of producing useful anti-idiotypes at the second step.

Step 3.) Compounding the anti-idiotypic vaccine and determining the optimal dosage:

If the intended recipient of the anti-idiotypic vaccine is a New Zealand White rabbit, each of the battery of purified anti-idiotypic monoclonal antibodies is combined with adjuvant and its antigenic potential is assessed with New Zealand White rabbits. Each of the battery of anti-idiotypic monoclonal antibodies in PBS solution is emulsified in Freund's adjuvant (1:1) to yield a total volume of 0.5mL with varying amounts of antibody. For New Zealand White rabbits, 500 micrograms of antibody is typically the optimal amount of antibody to obtain a maximum immune response. However, the optimal dosage ranges over a relatively broad plateau. Each anti-idiotypic monoclonal antibody should be tested for its individual optimal dosage. The 0.5mL of emulsion, having varying amounts of antibody, is then injected into New Zealand White rabbits. Similar injections are repeated after two weeks. After an additional two weeks, the rabbits are bled from an ear vein; the blood is clotted; and the serum is separated by centrifugation at 3000 RPM for 20 minutes. The serum is then tested for anti-E. histolytica activity.

A heterogeneous enzyme immunoassay may be used to measure for anti-E. histolytica activity. The heterogeneous enzyme immunoassay may be similar to the assay described above in step 1. The microtiter plates are coated with E. histolytica extract (100-200ng/well); rabbit serum samples are dispensed into the wells of the coated microtiter plates and allowed to react with the immobilized E. histolytica extract. The presence of rabbit anti-E. histolytica activity is detected using HRP labeled goat anti-rabbit IgG according to the HRP assay described above in Step 1.

After the useful anti-idiotypic monoclonal antibodies have been identified and their optimal dosages determined for the New Zealand White rabbit, each of the useful anti-idiotypic monoclonal antibodies may be compounded with the other such useful anti-idiotypic monoclonal antibodies to formulate an anti-idiotypic vaccine having an even greater antigenic potential than the individual components. The optimal dosage for each anti-idiotype within the compound is not necessarily identical to the optimal dosage for that anti-idiotype when administered individually. Synergistic and inhibitory effects may occur. The dosage of each component of the anti-idiotypic compound must then be optimized in relation to the dosage of every other component. One method to optimize the antigenic potential of the combination is to vary each component while holding the others constant and arriving at an optimum by a process of successive approximations. When optimizing the dosages by this process of successive approximations, the starting dosage of each component within the compound should be identical to the optimal dosage of that component when administered individually.

## Example B

A Method for Producing an Anti-idiotypic Vaccine against
Dirofilaria immitus Employing a Mixture of
Monoclonal Anti-idiotypic Fab Fragments

Step 1.) Production of a battery of idiotypic monclonal antibodies, each having a specificity directed against Dirofilaria immitus:

A battery of Murine idiotypic monoclonal antibodies recognizing a soluble protein antigen purified from D. immitus is prepared according to a procedure analogous to that described in Step 1 for making the anti-idiotypic vaccine against E. histolytica.

The soluble protein antigen from D. immitus is prepared as follows: Dirofilaria immitus (0.42g), mixed males and females, are homogenized in 25mL PBA for two minutes at 15 degrees Centigrade. The homogenate is then stored overnight at 4 degrees Centigrade. The homogenate is then pelleted by centrifugation. The supernatant is decanted and acidified by addition of 10% trichloroacetic acid (TCA) to pH 3.5 at 15 degrees Centigrade. If the acidity goes below pH 3.5, it may be necessary to back titrate by addition of 1N sodium hydroxide to return the pH to 3.5. The acidified supernatant is again centrifuged to remove precipitated material and then the remaining soluble protein in the supernatant is dialyzed exhaustively against buffer at 4 degrees Centigrade. This soluble protein can serve as antigenic material to elicit anti-D. immitus activity in an immune responsive subject.

BALB/c mice are injected with an emulsion of the protein antigen purified from D. immitus with 50 micrograms of protein. Otherwise, the protocol is analogous to the procedure for producing and purifying idiotypic monoclonal antibodies recognizing E. histolytica described above.

The heterogeneous immunoassay for anti-D. immitus activity is also analogous to the heterogeneous immunoassay for D. immitus. However, in the immunoassay for D. immitus, the microtiter plates are coated with the D. immitus antigen instead of the E. histolytica extract and HRP labeled rabbit anti-goat IgG was used, instead of goat anti-mouse IgG, to detect the binding of goat anti-D. immitus to polystyrene microtiter plates coated with D. immitus antigen (100-200ng/ml). Otherwise, the two assays are essentially the same.

Step 2.) Production of a battery of anti-idiotypic monoclonal antibodies, each having a specificity directed against the monoclonal anti-D. immitus antibodies:

The battery of anti-idiotypic monclonal antibodies recognizing monoclonal anti-D. immitus antibodies is prepared according to the procedure described in Step 2 for making the anti-idiotypic vaccine against E. histolytica. However, in this procedure, the BALB/c mice are immunized using emulsions made from each of the monoclonal anti-D. immitus antibodies isolated above. The procedures also differ because the antigen blocking control for detecting false positives in the heterogeneous immunoassay of the supernatant serum utilizes the D. immitus as the blocking

antigen instead of the E. histolytica extract. Otherwise the two procedures for producing and purifying a battery of anti-idiotypic monoclonal antibodies are essentially the same for E. histolytica and D. immitis.

Step 3.) Preparation of a battery of Fab fragments of anti-idiotypic monoclonal antibodies:

An aliquot of 150 milligrams of each anti-idiotypic monoclonal antibodies isolated and purified as described above is incubated at 37 degrees Centigrade for 16 hours, with mercuripapain (1.5 mg) in 0.1 molar phosphate buffer (pH 7.0), containing 0.01 molar cysteine and 0.002 molar ethylene-diamine tetra-acetate salt (EDTA) to activate the enzyme. After the incubation, further hydrolysis is stopped by oxidating and inactivating the enzyme by removing the cysteine and EDTA. The cysteine and EDTA can be removed by dialysing the resultant digest against several changes of water, with vigorous stirring, over a 48 hour period.

In order to separate out the digest products, the digest is then dialysed against 0.01 molar sodium acetate buffer (pH 5.5) and applied to a column (20 x 24 cm) of carboxymethyl (CM)-cellulose. After 200ml of eluate is collected, a gradient is applied to the column to remove the fragments. The gradient may consist of 1200mL of sodium acetate at pH 5.5, running from 0.1 to 0.9 molar. The elution profile from the column is followed by UV absorption at 280nm. Typically, the eluates will show three peaks together comprising 90 per cent of the total original Anti-Id. The first two peaks (Fractions I and II) contain Fab fragments; the third peak (Fraction III) contains Fc fragment. The Fab fragments from both Fractions I and II may be combined for each of the anti-idiotypic monoclonal antibodies to form a battery for use in compounding an anti-idiotypic vaccine.

Step 4.) Compounding the anti-idiotypic vaccine and determining the optimal dosage:

If the intended recipient of the anti-idiotypic vaccine is a goat, each of the battery of Fab fragments is combined with adjuvant and its antigenic potential is assessed by challenging goats with a subcutaneous injection of the anti-idiotypic vaccine followed by a second injection after two to four weeks to boost the immune response further. Each of the battery of Fab fragments, in PBS, is emulsified with Freund's adjuvant 1:1 to yield a total volume of 1mL with varying amounts of Fab fragments. For goats, 1000 micrograms of Fab fragments is typically the optimal amount to obtain a maximum immune response. However, the optimal dosage may range over a relatively broad plateau. The optimization of dosage of an anti-idiotypic vaccine composed of Fab fragments for eliciting an immune response in goats against E. histolytica is analogous to procedure for optimizing the dosage of an anti-idiotypic vaccine composed of intact anti-idiotypic monoclonal antibodies for eliciting an immune response in New Zealand White rabbits.

The presence of goat anti-D. imnitus antibodies in goat serum is determined by a heterogeneous immunoassay similar to that described above in step 1. The microtiter plates are coated with D. immitus antigen; the goat serum samples are dispensed into the coated wells and allowed to react with the immobilized D. immitus. The presence of goat anti-D. immitus activity is detected using HRP labeled rabbit anti-goat IgG according to the HRP assay described above in step 1 of Example A (E. histolytica).

The Fab fragments can be compounded in an emulsion and their component dosages optimized just as in the case of the anti-idiotypic vaccine against E. histolytica. Typically 1 to 10 Fabs, and preferably 1 to 5 Fabs, having different specificities can be compounded and used simultaneously to broaden the immune response. Both synergistic and inhibitory effects may occur as a result of such compounding. The optimal dosage of each component may be determined using a technique of sucessive approximations, varying each component while holding the others constant.

## CLAIMS

1. An anti-idiotypic vaccine for vaccinating an immune responsive subject so as to stimulate the subject to immunogenically produce a first idiotypic antibody having a first binding affinity with a first antigenic component comprising:

a carrier liquid, e.g. an oil-based adjuvant, and

a first purified anti-idiotypic monoclonal antibody or a fragment thereof, the antibody or fragment exhibiting structural congruence with at least one epitope of the first antigenic component,

said first anti-idiotypic antibody or fragment thereof being combined or emulsified in said carrier liquid for carrying a suggested dosage of said first anti-idiotypic antibody or fragment thereof when said anti-idiotypic vaccine is introduced into the subject,

the suggested dosage of said first anti-idiotypic antibody or fragment thereof having a first antigenic potential sufficient to stimulate the subject to immunogenically produce the first idiotypic antibody,

whereby said first idiotypic antibody immunogenically produced by the subject as a result of introducing the suggested dosage of said anti-idiotypic vaccine becomes available to the subject for responding immunologically against the first antigenic component.

2. An anti-idiotypic vaccine as in claim 1 wherein the first binding affinity between the first antigenic component and the first idiotypic antibody is sufficient to enable agglutination of an agglutinatable body when the first antigenic component is attached to the body.

3. An anti-idiotypic vaccine as in claim 1 wherein the suggested dosage of said first anti-idiotypic antibody or fragment thereof is optimized to maximize the first antigenic potential for stimulating the subject to immunogenically produce the first idiotypic antibody.

4. An anti-idiotypic vaccine for vaccinating an immune responsive subject so as to stimulate the subject to immunogenically produce a first idiotypic antibody having a first binding affinity with a first antigenic component and for stimulating the subject to immunogenically produce a second idiotypic antibody having a second binding affinity with a second antigenic component comprising:

a carrier fluid, which is preferably an oil-based adjuvant, and

a first purified anti-idiotypic monoclonal antibody or a fragment thereof, the antibody or fragment exhibiting structural congruence with at least one epitope of the first antigenic component,

a second purified anti-idiotypic monoclonal antibody or a fragment thereof, the antibody or fragment exhibiting structural congruence with at least one epitope of the second antigenic component,

said first anti-idiotypic antibody or fragment thereof and said second anti-idiotypic antibody or fragment thereof combined with said carrier fluid for carrying a suggested dosage of said first anti-idiotypic antibody or fragment thereof and said second anti-idiotypic antibody or fragment thereof when said anti-idiotypic vaccine is introduced into the subject,

the suggested dosage of said first anti-idiotypic antibody or fragment thereof having a first antigenic potential sufficient to stimulate the subject to immunogenically produce the first idiotypic antibody,

the suggested dosage of said second anti-idiotypic antibody or fragment thereof having a second antigenic potential sufficient to stimulate the subject to immunogenically produce the second idiotypic antibody,

whereby said first idiotypic antibody and said second idiotypic antibody immunogenically produced by the subject as a result of introducing the suggested dosage of said anti-idiotypic vaccine becomes available to the subject for responding immunologically against the first and second antigenic components.

5.   An anti-idiotypic vaccine as in claim 4 wherein the first binding affinity between the first antigenic component and the first idiotypic antibody is sufficient to enable agglutination of an agglutinatable body when the first antigenic component is attached to the body and the second binding affinity between the second antigenic component and the second idiotypic antibody is sufficient to enable agglutination of the body when the second antigenic component is attached to the body.

6.   An anti-idiotypic vaccine as in claim 4 or 5, wherein the suggested dosage of said first anti-idiotypic antibody or fragment thereof is optimized to maximize the first antigenic potential for stimulating the subject to immunogenically produce the first idiotypic antibody and the suggested dosage of said second anti-idiotypic antibody or fragment thereof is optimized to maximize the second antigenic potential for stimulating the subject to immunogenically produce the second idiotypic antibody.

7. An anti-idiotypic vaccine as in any one of claims 1 to 6 which includes one or more anti-idiotypic antibody fragments, wherein the or at least one fragment is a Fab fragment of a respective anti-idiotypic antibody.

8. A method for making an anti-idiotypic vaccine for vaccinating an immune responsive subject so as to stimulate the subject to immunogenically produce a first idiotypic antibody having a first binding affinity with a first antigenic component comprising the following steps:

producing a first anti-idiotypic monoclonal antibody exhibiting structural congruence with at least one epitope of the first antigenic component, then

purifying the first anti-idiotypic monoclonal antibody,

optionally isolating a fragment of the first anti-idiotypic monoclonal antibody exhibiting structural congruence with at least one epitope of the first antigenic component, and then

dispersing the first purified anti-idiotypic monoclonal antibody or the fragment with a carrier liquid, preferably by emulsifying it in an oil-based adjuvant.

9. A method as in claim 8 further comprising the step of:

38.

determining an optimal dosage of the vaccine which has a first antigenic potential sufficient for maximally stimulating the subject to immunogenically produce the first idiotypic antibody when the dosage is introduced into the subject.

10. A method as in claim 8 or claim 9, wherein the first binding affinity between the first antigenic component and the first idiotypic antibody is sufficient to enable agglutination of an agglutinatable body when the first antigenic component is attached to the body.

11. A method as in any one of claims 8 to 10 for additionally stimulating the subject to immunogenically produce a second idiotypic antibody having a second binding affinity with a second antigenic component further comprising the following steps:

producing a second anti-idiotypic monoclonal antibody exhibiting structural congruence with at least one epitope of the second antigenic component, then

purifying the second anti-idiotypic monoclonal antibody,

optionally isolating a fragment of the second anti-idiotypic monoclonal antibody exhibiting structural congruence with at least one epitope of the second antigenic component, then

dispersing the second purified anti-idiotypic monoclonal antibody or fragment thereof in the carrier liquid, preferably an oil based adjuvant and

mixing the first anti-idiotypic monoclonal antibody or fragment thereof and second anti-idiotypic monoclonal antibody or fragment thereof within the carrier liquid.